Europäisches Patentamt

European Patent Office

Office européen des brevets

(19)

(11) EP 0 680 941 B1

(12) FASCICULE DE BREVET EUROPEEN

(45) Date de publication et mention
de la délivrance du brevet:
**31.03.1999 Bulletin 1999/13**

(51) Int. Cl.$^6$: **C07C 2/58**
// B01J38/56

(21) Numéro de dépôt: **95400963.5**

(22) Date de dépôt: **27.04.1995**

(54) **Procédé d'alkylation de paraffines**

Verfahren zur Alkylierung von Paraffinen

Process for the alkylation of paraffins

(84) Etats contractants désignés:
**DE GB IT NL**

(30) Priorité: **05.05.1994 FR 9404925**

(43) Date de publication de la demande:
**08.11.1995 Bulletin 1995/45**

(73) Titulaire:
**INSTITUT FRANCAIS DU PETROLE
92502 Rueil-Malmaison (FR)**

(72) Inventeurs:
 • **Joly, Jean-François
   F-69006 Lyon (FR)**
 • **Courtly, Philippe
   F-78800 Houilles (FR)**
 • **Benazzi, Eric
   F-78360 Montesson (FR)**
 • **Euzen, Jean-Paul
   F-69570 Dardilly (FR)**
 • **Forestière, Alain
   F-69390 Vernaison (FR)**

(56) Documents cités:
   EP-A- 0 584 006          WO-A-93/10065
   US-A- 3 879 488          US-A- 5 157 196

## Description

La présente invention concerne un procédé d'alkylation d'au moins une isoparaffine, de préférence l'isobutane, par au moins une oléfine contenant de 3 à 6 atomes de carbone par molécule.

On sait que, pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation des isoparaffines (isobutane et/ou isopentane) par les oléfines contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Les procédés existant pour la production d'hydrocarbures par alkylation de l'isobutane par les oléfines utilisent soit l'acide sulfurique soit l'acide fluorhydrique comme catalyseur. Dans ces procédés le catalyseur acide constitue une phase liquide qui est mise en contact avec le mélange isobutane-oléfine liquide pour former une émulsion. Ces procédés sont coûteux et posent d'importants problèmes vis-à-vis de la sécurité des personnes et de l'environnement. Afin de remédier à ces problèmes, des systèmes catalytiques différents des acides sulfurique et fluorhydrique en phase liquide ont été recherchés.

La présente invention concerne p. ex. un procédé d'alkylation catalytique d'isobutane et/ou d'isopentane au moyen d'une oléfine, qui permet d'obtenir au moins un produit par exemple appartenant au groupe constitué par les diméthylbutanes, les triméthylpentanes, les triméthylhexanes et les triméthylheptanes, et où l'oléfine et/ou un mélange d'oléfines est introduit(e) dans le réacteur en phase liquide et en mélange avec l'isoparaffine et/ou un mélange d'isoparaffines.

Le procédé selon la présente invention permet de réaliser dans les meilleures conditions, et en particulier d'obtenir une bonne homogénéité de température et de concentration en réactifs, la réaction d'alkylation de l'isobutane par les oléfines, réaction qui est caractérisée par une forte exothermicité d'environ 20 kcal/M (83,70 kj) de butène transformé.

Dans le procédé d'alkylation de l'isobutane selon la présente invention, les conditions opératoires, et plus particulièrement la température et la pression, sont choisies de façon à ce que le mélange constitué par l'isobutane, les oléfines et les produits de la réaction soit liquide. De plus, il est important que le catalyseur baigne dans ledit liquide de façon à assurer partout un bon contact liquide-solide. Ce faisant on évite l'apparition de zones sèches dans le réacteur, lesquelles zones pourraient être responsables d'un manque de stabilité thermique, ces zones sèches pouvant atteindre des températures élevées-du fait de la réaction qui peut se dérouler entièrement en phase gazeuse à ces endroits. Aussi a-t-on proposé diverses techniques, utilisant une phase liquide continue, le catalyseur pouvant être utilisé sous forme de suspension, sous forme de lit bouillonnant ou sous forme de lit fixe ou mobile.

Plusieurs brevets revendiquent la mise en oeuvre de catalyseurs hétérogènes (alumine associée à $BF_3$ par exemple) dans des réacteurs agités du type Grignard (WO 92/03395, US-A-4918255, US-A-3655813). Cette mise en oeuvre du catalyseur présente plusieurs inconvénients, par exemple la destruction du catalyseur par attrition au cours du temps, de plus l'obtention de temps de séjour courts des hydrocarbures dans le réacteur est difficile à réaliser.

L'utilisation en lit(s) fixe(s) de catalyseurs hétérogènes d'alkylation est décrite dans les brevets EP-A-0433954, US-A-3852371 et US-A-3976713. L'obtention d'une dilution de l'oléfine très élevée au voisinage des grains de catalyseur est difficile, de plus l'utilisation de catalyseurs présentant une faible granulométrie s'accompagne de pertes de charges importantes.

Le brevet US-A-5157196 revendique l'utilisation de catalyseurs d'alkylation en lit circulant, ladite invention est en particulier caractérisée en ce qu'une zone de lavage du catalyseur est présente et fonctionne en lit fluide ; de plus, le temps de séjour des hydrocarbures dans le réacteur est court (1 seconde à 5 minutes).

La présente invention est basée sur un lavage perfectionné du catalyseur notamment par rapport au procédé du brevet US-A-5157196.

En effet le liquide de lavage décrit dans le procédé US-A-5157196, reste souillé lorsqu'il repart dans la zone réactionnelle, avec pour effet de re-souiller le catalyseur dans le réacteur.

La présente invention, concernant un procédé d'alkylation catalytique d'au moins une isoparaffine par au moins une oléfine contenant 3 à 6 atomes de carbone, comme defini dans les revendications, résout résout ce problème avantageusement.

Le procédé de la présente invention s'applique à tout catalyseur solide permettant de réaliser l'alkylation d'isoparaffine, et plus particulièrement de l'isobutane et/ou de l'isopentane, par des oléfines comprenant de 3 à 6 atomes de carbone par molécule.

Le catalyseur présent dans la zone R est choisi parmi les catalyseurs solides connus de l'homme du métier. De préférence, le catalyseur est choisi parmi les catalyseurs suivants :

* un catalyseur comprenant au moins de l'acide sulfurique imprégné sur un support poreux organique ou minéral, tels les catalyseurs décrits dans les demandes de brevet français 2682891, 2683740, 2683739 et 2687935,

* un catalyseur comprenant le mélange contenant d'une part au moins un halogénure de composé choisi dans le groupe formé par l'aluminium et le bore et d'autre part au moins un halogénure d'ammonium quaternaire et/ou halohydrate d'amine, tel par exemple que le catalyseur décrit dans la demande de brevet français 2686526.

Le catalyseur utilisé dans la présente invention, renferme de préférence, de la silice et de l'acide sulfurique, la silice étant imprégnée totalement par l'acide sulfurique. La silice est caractérisée en ce que son volume poreux total soit supérieur à 0,5 cm$^3$/g. Le catalyseur obtenu après imprégnation est caractérisé en ce que la teneur pondérale en acide sulfurique est supérieure à 45%, et de préférence supérieur à 75%.

La silice peut contenir des impuretés comme par exemple les oxydes, les alcalins, les alcalino-terreux, les composés d'aluminium ou toute autre impureté connue de l'homme du métier, la quantité totale de ces impuretés n'excédant pas 2 % en poids par rapport à la silice.

La concentration de l'acide sulfurique est avantageusement comprise entre 90 et 100 % en poids, de préférence entre 97 et 100 % en poids et de manière encore plus préférée entre 98 et 100 % en poids.

Il est possible d'ajouter dans la phase acide H$_2$SO$_4$, avant l'imprégnation, des additifs visant à améliorer les performances catalytiques. Parmi ces additifs on peut citer à titre d'exemple les acides trifluorométhanesulfonique CF$_3$SO$_3$H et HB(SO$_4$H)$_4$, et de préférence l'acide borique BO$_4$H$_3$ ou l'anhydride borique. Les catalyseurs préférés utilisés pour la présence invention (du type acide sulfurique sur silice, dopé de préférence par un composé du bore) donnent des résultats supérieurs à ceux que l'on obtiendrait avec des catalyseurs plus conventionnels du type silice dopée par SbF$_3$ ou SbF$_5$.

Le diamètre moyen des particules du catalyseur, principalement constitué de grains sensiblement sphériques, utilisé dans le procédé selon la présente invention est compris entre 0.1 et 200 microns (1 micron = 10$^{-6}$ m), de préférence entre 10 à 80 microns et d'une façon encore plus préférée entre 10 et 60 microns.

Ainsi l'invention concerne un procédé d'alkylation catalytique d'au moins une isoparaffine par au moins une oléfine contenant 3 à 6 atomes de carbone par molécule dans une zone réactionnelle en présence d'un lit circulant de catalyseur solide, qui circule à co-courant de la charge et de bas en haut de la zone réactionnelle R; procédé dans lequel :

a) on soutire de la zone réactionnelle R une partie au moins du catalyseur en mélange avec l'effluent réactionnel,

b) on sépare le catalyseur de l'effluent réactionnel, au moyen d'une zone de désengagement D,

c) on fractionne l'effluent réactionnel en au moins un alkylat et un fraction riche en isoparaffine, dans une zone de séparation S,

d) on lave le catalyseur dans une zone de lavage par une partie au moins de ladite fraction riche en isoparaffine, ladite partie circulant à contre-courant du catalyseur,

e) on renvoie une partie au moins du catalyseur lavé dans la zone réactionnelle.

f) on procède au nettoyage de l'effluent liquide riche en isoparaffine qui a servi à laver le catalyseur, par exemple à travers au moins un lit fixe (constitué généralement d'argile, d'alumine ou de bauxite),

g) on réunit dans une zone de réception C l'effluent liquide nettoyé riche en isoparaffine à l'étape f et le catalyseur lavé à l'étape d en créant dans la zone de réception C une suspension catalyseur-hydrocarbure(s), laquelle est envoyée au moins en partie dans la zone réactionnelle.

Dans un mode de mise en oeuvre, le procédé selon la présente invention est caractérisé en ce que :

- on soutire généralement en continu un effluent liquide, contenant en suspension au moins une partie du catalyseur, hors de la zone réactionnelle R,

- on recycle une partie de l'effluent liquide sortant de la zone de désengagement du catalyseur (D) vers l'entrée de ladite zone R,

- on recycle une partie de l'effluent liquide riche en isoparaffine de la zone S en zone R (de préférence à l'entrée de la zone R),

Selon une mise en oeuvre du procédé selon l'invention, il est possible d'introduire la charge, c'est-à-dire le composé décrit en a), en plusieurs points de la zone R. Ces différents points d'injection du composé sont répartis le long de la zone réactionnelle, et un de ces points d'injection est l'entrée de ladite zone. Ladite répartition se fait de façon la plus avantageuse pour le déroulement de la réaction, selon les conditions opératoires et les composés présents dans la zone R.

La température dans la zone réactionnelle R est généralement comprise entre -15 et +6°C, de préférence entre -15 et 0°C, et la pression est telle que tout produit injecté dans la zone R, est liquide à l'injection dans ladite zone.

L'élimination des calories sera réalisée d'une manière préférée par un ou plusieurs échangeurs de chaleur situés sur le circuit d'alimentation de la zone R, en sortie de la zone R et sur le circuit d'alimentation de la zone de lavage L, de façon à ce que la température du fluide en contact avec le catalyseur reste inférieure à +6°C et de préférence inférieure à 0°C.

De préférence, la charge a été séchée sur tamis moléculaire et hydrogénée sélectivement avant son introduction dans la zone réactionnelle R de manière à éliminer les composés très fortement insaturés susceptibles d'inhiber la phase catalytique.

Le rapport des débits massiques de la partie de l'effluent liquide sortant de la zone D recyclée à l'entrée de la zone R est généralement compris entre 1 et 10, de préférence entre 1 et 5.

Les réactifs sont introduits de façon à ce que la vitesse spatiale horaire, exprimée en poids d'oléfine(s) introduite(s) par unité de poids du catalyseur présent dans la zone R et par heure, soit généralement comprise entre 0,01 et 10 $h^{-1}$, de préférence entre 0,02 et 2 $h^{-1}$, et de manière encore plus préférée entre 0,025 et 1 $h^{-1}$.

La concentration volumique de catalyseur dans la zone réactionnelle R, exprimée en volume de catalyseur par volume de phase liquide hydrocarbure est comprise entre 1:100 et 1:1 et de préférence entre 1:100 et 1:50, et d'une façon encore plus préférée entre 1:50 et 1:4.

Afin de limiter les réactions secondaires, on utilise un excès d'isoparaffine par rapport à l'oléfine à l'entrée de la zone réactionnelle R. A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butènes à l'entrée de la zone réactionnelle R est généralement compris entre 1 et 1000, de préférence entre 10 et 500 et de manière souvent préférée entre 15 et 200.

De façon à limiter les réactions secondaires de dégradation des isoparaffines $C_5$-$C_{12}$ présentes dans les effluents liquides passant au travers de(s) zone(s) de réactions R, on réalise l'opération de séparation S de façon à ce que le rapport des débits massiques d'isoparaffine (par exemple d'isobutane) en tête de la zone de séparation S et d'alkylat en fond de la zone S soit compris entre 5:1 et 100:1, et de préférence compris entre 10:1 et 30:1.

Un ou plusieurs réacteurs peuvent être utilisés dans le procédé selon l'invention. Dans le cas ou on utilise plusieurs réacteurs en série, il est avantageux d'introduire l'oléfine à l'entrée de chacun des réacteurs, et d'introduire l'effluent liquide constitué par la somme des effluents et l'isobutane de la charge en majeure partie à l'entrée du premier réacteur. De plus l'effluent sortant de chaque réacteur passe au travers d'un échangeur avant d'être introduit à l'entrée du réacteur suivant.

Dans une réalisation préférée du procédé selon la présente invention on soutire en continu du catalyseur désactivé et on introduit du catalyseur frais ou du catalyseur régénéré dans le récipient C. Les quantités de catalyseur soutirées étant égales aux quantités de catalyseurs introduites.

La figure jointe illustre l'invention, et plus précisément une des mises en oeuvre préférées du procédé selon l'invention, sans en limiter la portée.

Le mélange en phase liquide de la ligne (3), comprenant au moins une isoparaffine, (de préférence au moins une isoparaffine choisie dans le groupe formé par l'isobutane et l'isopentane) amené par la ligne (1), au moins une oléfine contenant de 3 à 6 atomes de carbone par molécule amenée par la ligne (2), est introduit par la ligne (4) dans la zone réactionnelle R, dans laquelle également est introduit par la ligne (7) un effluent liquide défini ci-dessous.

Cet effluent contient une suspension de catalyseur dans un mélange des effluents des lignes (21) et (5) et est donc introduit en bas de la zone réactionnelle R par la ligne (7).

Des échangeurs de chaleur $E_1$ et $E_2$, situés sur les lignes (4) et (7), permettent d'ajuster la température des effluents entrant dans la zone réactionnelle R.

L'effluent liquide contenant la suspension de catalyseur sortant de la zone réactionnelle R par la ligne (8) passe au travers d'un échangeur de chaleur $E_3$ qui permet d'éliminer la chaleur de la rection d'alkylation. Le catalyseur est ensuite séparé de l'effluent liquide de la ligne (8) au moyen d'une zone de désengagement D. L'effluent liquide ne contenant plus de catalyseur est envoyé par la ligne (9) puis par la ligne (17) vers la zone de séparation S. Une partie de l'effluent de la ligne (9) est recyclée, par les lignes (5) avec pompe P1 puis (7) à l'entrée de la zone de réaction R. Le catalyseur séparé de l'effluent de la ligne (8) est introduit par la ligne (10) dans une zone de lavage L, où circule, à contre-courant du catalyseur, une partie de l'effluent provenant de la tête de la zone de séparation S par les lignes (19) puis (11). Le catalyseur sort de la zone de lavage L par le bas et est introduit par la ligne (14) dans un bac de remise en suspension C. L'effluent liquide sortant du haut de la zone de lavage L par la ligne (12) passe au travers d'une zone de neutralisation N puis est introduit, par la ligne (13) dans le bac de remise en suspension C du catalyseur. La suspension de catalyseur sortant du bac de remise en suspension C est introduit par la ligne (21), (avec pompe P2 et échangeur E4) par la ligne (7) après mélange avec l'effluent de la ligne (5) à l'entrée de la zone réactionnelle R.

Du catalyseur usé peut être extrait de l'unité par exemple par la ligne (15a), l'appoint de catalyseur neuf nécessaire pour maintenir la productivité de l'unité est introduit dans le bac de remise en suspension C par la ligne (16).

La partie de l'effluent liquide de la ligne (9) qui n'est pas recyclée par la ligne (5) vers l'entrée de ladite zone R, est envoyée par la ligne (17) vers une zone de séparation isobutane/normal-butane/alkylat (S). L'alkylat séparé dans la zone S est extrait de l'unité à titre de produit par la ligne (20). Le normal butane est extrait latéralement de la zone S par la ligne (18) à titre de purge. La fraction liquide riche en isobutane extraite en tête de la zone S est recyclée vers l'entrée de la zone réactionnelle R par les lignes (19) puis (6) et vers la zone de lavage L par les lignes (19) puis (11).

L'exemple suivant illustre l'invention.

Exemple 1

Composition du catalyseur

Le catalyseur comprend 20% en poids d'une silice de diamètre moyen des particules égal à 60 microns et 80% en poids d'un acide sulfurique de titre voisin de 100% poids.

Alkylation de l'isobutane par une coupe butènes

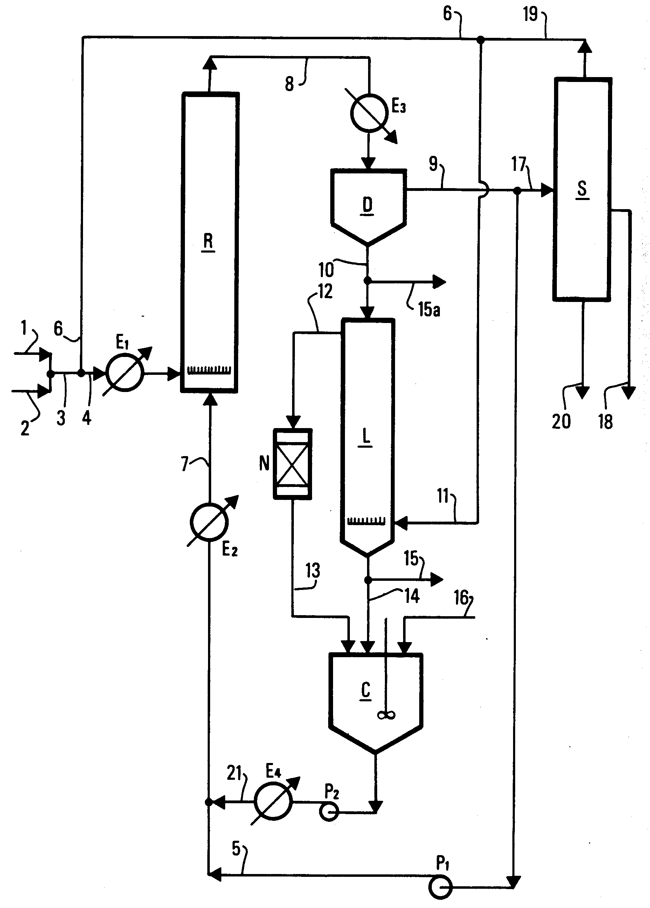

On réalise l'alkylation de l'isobutane par les butènes (mélange contenant 45% en poids de butène-1 et 55% en poids de butènes 2 cis et trans) au moyen du procédé selon l'invention, et en particulier selon la mise en oeuvre préférée illustrée par la figure 1.

61 tonnes de catalyseur sont utilisées pour produire 12,5 t/h d'alkylat, ce qui correspond à une production annuelle de 100000 t d'alkylat.

Le catalyseur circule entre une zone de réaction R, composée de deux réacteurs en série et une zone de lavage L. La vitesse de circulation de la suspension de catalyseur est voisine de 2,5 cm/s dans les deux réacteurs, le temps de séjour du catalyseur dans chacun des deux réacteurs est égal à environ 5 minutes. Le débit massique de liquide à la sortie du second réacteur est égal à 600 t/h. Le catalyseur est séparé de l'effluent liquide au moyen de cyclones (zone de séparation S), puis passe à contre courant d'une phase liquide riche en isobutane dans la zone de lavage. Le débit massique d'isobutane dans cette zone de lavage est de 6,2 t/h, la vitesse de circulation du catalyseur (de haut en bas) dans le zone de lavage est voisine de 0,5 cm/s.

La température des effluents liquides à l'entrée des deux réacteurs est égale à -7,5°C. La température dans la zone de lavage est voisine de -5°C.

La coupe butènes de la charge est introduite en deux parts égales à l'entrée de chacun des deux réacteurs à des débits massiques égaux à 3,07 t/h. L'isobutane de la charge est introduit en totalité à l'entrée du premier réacteur à un débit massique égal à 6,4 t/h.

Une partie de l'effluent liquide sortant de la zone de désengagement D, est recyclée vers l'entrée du premier réacteur. Le débit massique de recycle est égal à 400 t/h. L'autre partie de l'effluent liquide sortant de la zone de désengagement D est envoyée dans la zone de fractionnement isobutane-alkylat à un débit massique de 200 t/h. On soutire en fond de la colonne de l'alkylat à un débit de 12,5 t/h et de l'isobutane en tête de colonne à un débit de 187,5 t/h. La majeure partie de l'isobutane est recyclé à l'entrée du premier réacteur à un débit de 181,3 t/h, le reste soit 6,2 t/h est envoyé vers la zone de lavage L où il circule à contre-courant du catalyseur à laver. Le catalyseur lavé et l'isobutane de lavage (après passage de ce dernier sur un lit d'argile) sont réunis et envoyés au réacteur.

L'alkylat produit à la composition pondérale suivante :

| | |
|---|---|
| $C_5$-$C_7$ | 4,67 |
| $C_8$ | 89,31 |
| $C_9^+$ | 6,02 |

La fraction $C_8$ contient 93% en poids de triméthylpentanes. L'indice d'octane (R+M)/2 de l'alkylat est égal à 97.

**Revendications**

1. Procédé d' alkylation catalytique d'au moins une isoparaffine par au moins une oléfine contenant 3 à 6 atomes de carbone par molécule dans une zone réactionnelle en présence d'un lit circulant de catalyseur solide, oui circule à co-courant de la charge et de bas en haut de la zone réactionnelle R ; procédé dans lequel :

a) on soutire une partie au moins du catalyseur en mélange avec l'effluent réactionnel,

b) on sépare le catalyseur de l'effluent réactionnel, au moyen d'une zone de désengagement D,

c) on fractionne l'effluent réactionnel en au moins un alkylat et une fraction riche en isoparaffine, dans une zone de séparation S,

d) on lave le catalyseur dans une zone de lavage par une partie au moins de ladite fraction riche en isoparaffine, ladite partie circulant à contre-courant du catalyseur,

e) on renvoie une partie au moins du catalyseur lavé vers la zone réactionnelle, ledit procédé étant caractérisé en ce que :

f) on procède au nettoyage de l 'effluent liquide riche en isoparaffine qui a servi à laver le catalyseur, et

g) on réunit dans une zone de réception C l'effluent liquide nettoyé riche en isoparaffine à l'étape (f) et le catalyseur lavé à l'étape (d) en créant dans la zone de réception C une suspension catalyseur-hydrocarbure(s), laquelle est envoyée au moins en partie dans la zone réactionnelle.

2. Procédé selon la revendication 1 dans lequel le catalyseur est soutiré en continu de la zone réactionnelle en suspension dans l'effluent liquide.

3. procédé selon l'une des revendications 1 à 2 dans lequel la charge est introduite en plusieurs points de la zone réactionnelle à des niveaux différents.

4. Procédé selon l'une des revendications 1 à 3 dans lequel on recycle une partie de l 'effluent liquide sortant de la zone de désengagement du catalyseur D vers l'entrée de la zone réactionnelle R.

5. Procédé selon l'une des revendications 1 à 4 dans lequel on recycle une partie de la fraction riche en isoparaffine de la zone S en zone R.

6. Procédé selon l'une des revendications 1 à 5 dans lequel l'isoparaffine est choisie dans le groupe formé par l'isobutane et l'isopentane.

7. Procédé selon l'une des revendications 1 à 6 dans lequel le rapport des débits massiques de la partie de l 'effluent liquide sortant de la zone D recyclée à l'entrée de la zone R et des mélanges de la charge, de la partie de l 'effluent liquide riche en isoparaffine recyclée de la zone S dans la zone réactionnelle R et de la suspension créée dans la zone de réception C est compris entre 1 et 10.

8. Procédé selon l'une des revendications 1 à 7 dans lequel la concentration volumique de catalyseur dans la zone réactionnelle R, exprimée en volume de catalyseur par volume de phase liquide hydrocarbure, est comprise entre 1:100 et 1:1.

9. Procédé selon l'une des revendications 1 à 8 dans lequel on réalise l'opération de séparation S de façon à ce que le rapport des débits massiques d'isoparaffine en tête de la zone de séparation S et d' alkylat en fond de la zone de séparation S soit compris entre 5:1 et 100:1.

10. Procédé selon l'une des revendications 1 à 9 tel que le diamètre moyen des particules du catalyseur, principalement constitué de grains sensiblement sphériques, est compris entre 0,1 et 200.10⁻⁶m.

11. Procédé selon l'une des revendications 1 à 10 tel que les réactifs sont introduits de façon à ce que la vitesse spatiale horaire exprimée en poids d'oléfine(s) introduite(s) par unité de poids de catalyseur présent dans la zone R et par heure est compris entre 0,1 et 10 h et tel que la température de la zone réactionnelle R est comprise entre -15 et +6° C.

**Claims**

1. A process for the catalytic alkylation of at least one isoparaffin by at least one olefin containing 3 to 6 carbon atoms per molecule in a reaction zone in the presence of a circulating bed of a solid catalyst which circulates as a co-current with the feed and from bottom to top in the reaction zone R; in which process:

   a) at least a portion of the catalyst is extracted mixed with the reaction effluent;
   b) the catalyst is separated from the reaction effluent in a release zone D;
   c) the reaction effluent is tractionated in a separation zone S into at least one alkylate and an isoparaffin-rich fraction;
   d) the catalyst is washed in a washing zone by at least a portion of said isoparaffin-rich traction, said portion circulating as a counter-current to the catalyst;
   e) at least a portion of the washed catalyst is returned to the reaction zone: said process being characterized in that:
   f) the isoparaffin-rich liquid effluent which has washed the catalyst is cleaned; and
   g) the cleaned isoparaffin-rich liquid effluent from step (f) is combined with the washed catalyst from step (d) in a receiving zone C to form a catalyst-hydrocarbon(s) suspension in receiving zone C, at least a portion of said suspension being sent to the reaction zone.

2. A process according to claim 1 in which the catalyst is continuously extracted from the reaction zone, in suspension in the liquid effluent.

3. A process according to claim 1 or claim 2, in which the feed is introduced into the reaction zone at a plurality of points at different levels.

4. A process according to any one of claims 1 to 3, in which a portion of the liquid effluent leaving the catalyst release zone D is recycled to the inlet to reaction zone R.

5. A process according to any one of claims 1 to 4, in which a portion of the isoparaffin-rich fraction is recycled from zone S to zone R.

6. A process according to any one of claims 1 to 5, in which the isoparaffin is selected from the group formed by isobutane and isopentane.

7. A process according to any one of claims 1 to 6, in which the ratio of the mass flow rates of the portion of liquid effluent leaving zone D recycled to the inlet to zone R to the feed mixtures, and of the portion of isoparaffinrich liquid effluent recycled from zone S to reaction zone R to the suspension created in receiving zone C is in the range 1 to 10.

8. A process according to any one of claims 1 to 7, in which the concentration of catalyst in reaction zone R, expressed as the volume of catalyst per volume of liquid hydrocarbon phase, is in the range 1:100 to 1:1 by volume.

9. A process according to any one of claims 1 to 8, in which the separation operation S is carried out such that the ratio of the mass flow rates of isoparaffin at the head of the separation zone S and alkylate at the bottom of the separation zone S is in the range 5:1 to 100:1.

10. A process according to any one of claims 1 to 9, in which the average diameter of the catalyst particles, principally constituted by substantially spherical grains, is in the range 0.1 to 200 x $10^{-6}$m.

11. A process according to any one of claims 1 to 10, in which the reactants are introduced such that the hourly space velocity, expressed as the weight of olefin(s) introduced per unit weight of catalyst present in zone R per hour, is in the range 0.1 to 10 $h^{-1}$ and such that the temperature in reaction zone R is in the range -15°C to +6°C.

**Patentansprüche**

1. Verfahren zur katalytischen Alkylierung mindestens eines Isoparaffins mit mindestens einem Olefin, das 3 bis 6 Kohlenstoffatome pro Molekül enthält, in einer Reaktionszone in Gegenwart eines zirkulierenden Feststoff-Katalysator-Bettes, das im Gegenstrom zur Beschickung und von unten nach oben in der Reaktionszone R zirkuliert, wobei das Verfahren umfaßt:

    a) das Abziehen mindestens eines Teils des Katalysators im Gemisch mit dem Reaktionsabstrom,
    b) die Trennung des Katalysators von dem Reaktionsabstrom in einer Absitz- bzw. Sedimentationszone D,
    c) die Fraktionierung des Reaktionsabstromes unter Bildung mindestens eines Alkylats und einer an Isoparaffin reichen Fraktion in einer Trennzone S,
    d) das Waschen des Katalysators in einer Waschzone mit mindestens einem Teil der genannten, an Isoparaffin reichen Fraktion, wobei der genannte Teil im Gegenstrom zu dem Katalysator zirkuliert wird, und
    e) das Einführen mindestens eines Teils des gewaschenen Katalysators in die Reaktionszone,
    und wobei das genannte Verfahren dadurch gekennzeichnet ist, daß man
    f) den an Isoparaffin reichen flüssigen Abstrom, der zum Waschen des Katalysators verwendet worden ist, reinigt und
    g) in einer Empfangszone C den an Isoparaffin reichen flüssigen Abstrom, der in der Stufe (f) gereinigt worden ist, und den Katalysator, der in der Stufe (d) gewaschen worden ist, miteinander vereinigt unter Bildung in der Empfangszone C einer Katalysator-Kohlenwasserstoff(e)-Suspension, die mindestens zum Teil in die Reaktionszone eingeführt wird.

2. Verfahren nach Anspruch 1, bei dem der Katalysator in Form einer Suspension in dem flüssigen Abstrom kontinuierlich aus der Reaktionszone abgezogen wird.

3. Verfahren nach einem der Ansprüche 1 bis 2, bei dem die Beschickung an mehreren Punkten der Reaktionszone in unterschiedlichen Niveaus eingeführt wird.

4. Verfahren nach einem der Ansprüche 1 bis 3, bei dem ein Teil des flüssigen Abstroms, der die Absitz- bzw. Sedimentationszone des Katalysators D verläßt, in die Reaktionszone R recyclisiert wird.

5. Verfahren nach einem der Ansprüche 1 bis 4, bei dem man einen Teil der an Isoparaffin reichen Fraktion aus der Zone S in die Zone R recyclisiert.

6. Verfahren nach einem der Ansprüche 1 bis 5, bei dem man das Isoparaffin auswählt aus der Gruppe, die besteht aus Isobutan und Isopentan.

7. Verfahren nach einem der Ansprüche 1 bis 6, bei dem das Verhältnis der Massendurchsätze zwischen dem Teil des flüssigen Abstroms, der die Zone D verläßt und in die Zone R recyclisiert wird, und den Gemischen aus der Beschickung, dem Teil des an Isoparaffin reichen flüssigen Abstroms, der aus der Zone S in die Reaktionszone R recyclisiert worden ist, und der in der Empfangszone C erzeugten Suspension zwischen 1 und 10 liegt.

8. Verfahren nach einem der Ansprüche 1 bis 7, bei dem die Volumen-Konzentration des Katalysators in der Reaktionszone R, ausgedrückt in Volumenteilen Katalysator pro Volumenteil der flüssigen Kohlenwasserstoffphase, zwischen 1:100 und 1:1 liegt.

9. Verfahren nach einem der Ansprüche 1 bis 8, bei dem man die Abtrennung S in der Weise durchführt, daß das Verhältnis zwischen den Massendurchsätzen von Isoparaffin am Kopf der Trennzone S und von Alkylat am Boden der Trennzone S zwischen 5:1 und 100:1 liegt.

10. Verfahren nach einem der Ansprüche 1 bis 9, bei dem der mittlere Durchmesser der Teilchen des Katalysators, der hauptsächlich aus im wesentlichen kugelförmigen Körnchen besteht, zwischen 0,1 und $200.10^{-6}$ m liegt.

11. Verfahren nach einem der Ansprüche 1 bis 10, bei dem die Reagentien so eingeführt werden, daß die stündliche Raumgeschwindigkeit, ausgedrückt in Gewichtseinheiten an eingeführtem (eingeführten) Olefin (Olefinen) pro Gewichtseinheit des in der Zone R vorhandenen Katalysators und pro Stunde, zwischen 0,1 und 10 $h^{-1}$ liegt und daß die Temperatur der Reaktionszone R zwischen -15 und +6°C liegt.